# EUROPÄISCHE PATENTANMELDUNG

(11) **EP 2 026 577 A1**
(43) Veröffentlichungstag der Anmeldung: **18.02.2009**
(21) Anmeldenummer: 07015565.0
(22) Anmeldetag: 08.08.2007
(51) Int. Cl.: H04N 7/12, H04N 11/00, H04N 7/10, H04N 7/015, H04N 7/06, A61B 19/00, F21S 8/00

(54) **Verfahren zur Übertragung eines Videosignals einer an einem OP-Leuchtensystem angeordneten Kamera sowie OP-Leuchtensystem**

(71) Anmelder: TRUMPF Medizin Systeme GmbH + Co. KG, 82178 Pucheim (DE)
(72) Erfinder: Völker, Thomas, 63456 Hanau (DE)
(74) Vertreter: Feldmeier, Jürgen

(57) **Zusammenfassung**

Ein Verfahren zur Signalübertragung eines Videosignals, insbesondere eines HDTV-Signals, bestehend aus mehreren Signalkomponenten, über ein Übertragungssystem (13), wobei die Anzahl der Kanäle (13a, 13b) des Übertragungssystems (13) geringer ist als die Anzahl der Komponenten des Videosignals, umfasst die Verfahrensschritte:
a. Komprimieren zumindest einer Signalkomponente;
b. Übertragen der zumindest einen komprimierten Signalkomponente über einen Kanal (13a, 13b) des Übertragungssystems (13);
c. Wiederherstellen des Videosignals.

Dadurch kann ein HDTV-Signal über ein Tragsystem eines OP-Leuchtensystems übertragen werden.

## Beschreibung

Die Erfindung betrifft ein Verfahren zur Signalübertragung eines Videosignals, insbesondere eines HDTV-Signals, bestehend aus mehreren Signalkomponenten, über ein Übertragungssystem, wobei die Anzahl der Kanäle des Übertragungssystems geringer ist als die Anzahl der Komponenten des Videosignals. Die Erfindung betrifft außerdem ein Operations (OP)-Leuchtensystem mit einer Operationsleuchte und einer an einem OP-Leuchten-Tragsystem angeordneten Kamera, die ein Videosignal liefert, und einem mit der Kamera über ein Übertragungssystem verbundenen Kamerasteuergerät, wobei das Übertragungssystem in dem Tragsystem angeordnet ist.

HDTV-Kameras erzeugen ein so genanntes YUV-Komponentensignal. Das YUV-Komponentensignal verteilt die in einem Bild enthaltenen Informationen auf drei Kanäle. Der erste, der Y-Kanal, liefert die Helligkeitsinformation, bildet für sich alleine betrachtet also die Schwarz-Weiss-Information des Kamerasignals. Die beiden U- und V- Komponenten, auch Vektoren genannt, bilden durch ihre jeweiligen positiven oder negativen Spannungswerte die Lage des Farbpunktes im "Farbkreis" ab.

Die Signalübertragung des YUV-Komponentensignals einer HDTV-Kamera über ein OP-Leuchten-Tragsystem ist problematisch, da für eine analoge Übertragung die Anzahl der benötigten Kanäle (Leitungen) bei der Verwendung von Schleifkontakten, also bei Gelenken ohne Drehwinkelbegrenzung, zu groß ist. Das Übertragungssystem besteht aus zwei paarweise verseilten (verdrillten) Leitungspaaren, die an den Gelenken über Schleifringe, somit frei durchdrehbar, von Arm zu Arm verbunden sind. Die einzelnen Armsegmente des Tragsystems sind zwar in Form eines Rohres ausgeführt, durch das die Leitungen geführt sind, unter HF-technischen Gesichtspunkten sind die Arme speziell an den Verbindungen jedoch als "offen" anzusehen. Eine effektive Abschirmung von HF-Signalen besteht also nicht. Für eine digitale Übertragung sind sehr große Übertragungsfrequenzen erforderlich. Auf Grund der schwierigen HF-Abschirmung des Tragarmsystems ist diese Übertragungsart sehr störanfällig und die Gefahr einer unzulässig hohen Abstrahlung sehr groß.

Aus diesen Gründen ist eine digitale serielle Übertragung nicht möglich, da hier mit Taktfrequenzen im Bereich von 1,4 GHz übertragen würde, was sich aufgrund der Leitungsarten nicht realisieren lässt. Ein parallele Übertragung ist aufgrund der mangelnden Anzahl von Leitungen ebenfalls nicht möglich.

Aufgabe der vorliegenden Erfindung ist es, ein Verfahren und ein OP-Leuchtensystem bereit zu stellen, so dass die bestehenden Übertragungssysteme der Leuchteninstallation weiterhin verwendet werden können.

Gelöst wird diese Aufgabe erfindungsgemäß auf ebenso überraschende wie einfache Art und Weise durch ein Verfahren der eingangsgenannten Art, mit den Verfahrensschritten :
a. Komprimieren zumindest einer Signalkomponente;
b. Übertragen der zumindest einen komprimierten Signalkomponente über einen Kanal des Übertragungssystems;
c. Wiederherstellen des Videosignals. Dadurch ist es möglich, mehrere komprimierte Signale auf demselben Kanal zu übertragen. Somit ist eine Videosignalübertragung bei einer begrenzten Anzahl von Übertragungskanälen möglich.

Eine bevorzugte Verfahrensvariante sieht daher vor, dass zumindest zwei analoge Signalkomponenten komprimiert und über denselben Kanal übertragen werden. Durch die Verteilung der Farbkomponenten ergeben sich bei den Bandbreiten für die unterschiedlichen Signale auch unterschiedliche Werte. Die beiden Farbkomponenten sind aufgrund ihrer Gewinnung aus benachbarten Bildpunkten mit einer signifikant geringeren Bandbreite ausgestattet. Somit lässt sich bei der Übertragung eine zeitliche Kompression und nachfolgende Expansion durchführen, die, wenn die Gesamtstrecke mit hoher Bandbreite betrieben wird, ohne Folge für die Ursprungssignale bleiben kann. Es ist denkbar, mehr als zwei Signalkomponenten, beispielsweise 3 Signalkomponenten, zu komprimieren und über denselben Kanal zu übertragen.

Eine vorteilhafte Weiterbildung zeichnet sich dadurch aus, dass die komprimierten Signalkomponenten zeit-gemultiplext übertragen werden. Insbesondere können 2 oder 3 komprimierte Signalkomponenten auf diese Weise übertragen werden.

Gemäß einer Ausgestaltung des Verfahrens kann vorgesehen sein, dass die Signalkomponenten mit einer von der Anzahl der über denselben Kanal übertragenen Signalkomponenten abhängigen Geschwindigkeit an das Übertragungssystem ausgegeben werden. Beispielsweise können die U- und die V-Signalkomponenten in der jeweiligen Zeitachse mit der jeweils doppelten Geschwindigkeit ausgegeben werden. So kann in der Zeitdauer einer Zeilenübertragung in der ersten Hälfte der Zeit die erste Komponente und in der zweiten Hälfte der Zeitdauer die zweite Komponente, jeweils mit doppelter Geschwindigkeit, ausgegeben werden. Auf der Gegenseite der Übertragungsstrecke (des Übertragungssystems) ist nun die Regeneration der ursprünglichen Signale erforderlich. Hierzu werden die beiden Anteile der seriell in der Zeile enthaltenen Farbkomponentenanteile wieder derart aufbereitet, dass sie nun in einer weiteren Verarbeitungsstufe als 2 Signale, nun mit halber Geschwindigkeit, wieder ihrem ursprünglichen Signal entsprechen. Wenn entsprechend drei Signalkomponenten während der Zeitdauer einer Zeilenübertragung übertragen werden sollen, müssen sie mit der dreifachen Geschwindigkeit ausgegeben werden.

Besonders vorteilhaft ist es im Zusammenhang mit HDTV-Signalen, wenn eine Signalkomponente unkomprimiert über einen Kanal übertragen wird. Die höchste Bandbreite wird für die Y-Information benötigt. Diese Information wird für den Schärfeeindruck benötigt und sollte somit auch keine Beeinflussung erfahren. Diese Signalkomponente kann aufgrund der Kompression der anderen Komponenten alleine unkomprimiert über einen zweiten Kanal übertragen werden. Daher kann erfindungsgemäß das YUV-Komponentensignal auf nur zwei Kanäle aufgesplittet werden, so dass auf einem Kanal das Y-Signal differenziell analog übertragen wird und auf dem zweiten Kanal ein gemultiplextes UV-Signal übertragen wird. Am Ende der Signalverarbeitung liegt wieder ein normgerechtes 3-kanaliges YUV-Signal vor, welches die Basis für unterschiedliche Konvertierungen darstellen kann.

Gemäß einer alternativen Art der Komprimierung kann auch aus zwei Signalkomponenten ein komprimiertes analoges Videosignal generiert werden.

Bei einer alternativen Verfahrensvariante kann vorgesehen sein, dass das Videosignal bzw. seine Signalkomponenten digitalisiert werden und ein digitales komprimiertes Signal erzeugt wird, das über einen Kanal übertragen wird. Das HDTV-Signal kann in verschiedenen Formaten dargestellt werden. Hierbei sind beispielsweise analoge Drei-Kanal-Komponentendarstellungen sowie verschiedenartige digitale Formate in paralleler und serieller Form möglich. Wenn das HDTV-Signal in digitaler Form vorliegt oder in digitale Form gebracht wird, kann eine MPEG-Komprimierung erfolgen und das komprimierte Signal über nur einen Kanal übertragen werden.

In den Rahmen der Erfindung fällt außerdem ein OP-Leuchtensystem der eingangs genannten Art, wobei an der Kamera eine Einrichtung zur Komprimierung zumindest einer Signalkomponente des Videosignals vorgesehen ist. Durch diese Maßnahme ist es möglich, HDTV-Signale über ein Übertragungssystem mit weniger Kanälen als Signalkomponenten zu übertragen. Insbesondere ist es möglich, ein herkömmliches, bei OP-Leuchten bereits eingesetztes Übertragungssystem zu verwenden. Wenn in dem Übertragungssystem ein verdrilltes Leitungspaar vorgesehen ist, so stellt dies einen Kanal dar, über den ein Signal differentiell übertragen werden kann. In den Tragarmen ist vorzugsweise ein 7-poliges System angeordnet, wobei zwei Leitungen (Pole) zur Spannungsversorgung dienen und eine Leitung ein Schutzleiter ist. Die übrigen vier Leitungen sind paarweise verdrillt. Jedes Paar stellt einen Kanal dar.

Bei einer besonders bevorzugten Ausführungsform weist das Tragsystem zumindest zwei Tragarmabschnitte auf, die durchdrehbar miteinander verbunden sind. Dies bedeutet, dass eine Drehung um 360° oder mehr möglich ist, da kein Anschlag vorgesehen ist. Zu diesem Zweck kann an der Verbindungsstelle der Tragarmabschnitte ein Schleifringsystem vorgesehen sein, das die Datenübertragung und Stromversorgung auch über diese Verbindungsstelle hinweg erlaubt. Diese Verbindungsstelle kann nicht ohne weiteres für mehr als 2 Datenkanäle erweitert werden, weshalb ohne die vorliegende Erfindung die Übertragung eines HDTV-Signals nicht möglich wäre.

Die Kamera kann ein analoges HDTV-Signal, das drei Signalkomponenten aufweist, liefern. Diese Signalkomponenten können besonders einfach komprimiert und übertragen werden.

Vorteilhafterweise ist kameraseitig eine Zeitmultiplexeinrichtung vorgesehen. Dadurch können die komprimierten Signalkomponenten zeitversetzt an das Übertragungssystem gegeben werden und mehrere Signalkomponenten (in komprimierter Form) über denselben Kanal übertragen werden.

Um die ursprünglichen Signalkomponenten zu rekonstruieren ist vorteilhafterweise steuergeräteseitig eine Zeitdemultiplexeinrichtung vorgesehen.

Gemäß einer Ausgestaltung der Erfindung können kameraseitig ein A/D-Wandler und eine MPEG-Komprimiereinrichtung vorgesehen sein. Alternativ können gegebenenfalls vorhandene digitale Signale der Kamera direkt verwendet werden. Dies ermöglicht die Übertragung komprimierter digitaler Signale.

Weitere Merkmale und Vorteile der Erfindung ergeben sich aus der nachfolgenden Beschreibung von Ausführungsbeispielen der Erfindung, anhand der Figuren der Zeichnung, die erfindungswesentliche Einzelheiten zeigen, und aus den Ansprüchen. Die einzelnen Merkmale können je einzeln für sich oder zu mehreren in beliebiger Kombination bei einer Variante der Erfindung verwirklicht sein.

Bevorzugte Ausführungsbeispiele der Erfindung sind in der Zeichnung schematisch dargestellt und werden nachfolgend mit Bezug zu den Figuren der Zeichnung näher erläutert. Es zeigt:
- **Fig. 1a**: eine erste Prinzipdarstellung eines OP-Leuchtensystems;
- **Fig. 1b**: eine zweite Prinzipdarstellung eines OP-Leuchtensystems;

- **Fig. 2**: einen Ausschnitt einer ersten Ausführungsform eines OP-Leuchtensystem mit einer von Leuchtmitteln umgebenen Kamera;
- **Fig. 3**: einen Ausschnitt einer zweiten Ausführungsform eines OP-Leuchtensystem mit einer neben der Operationsleuchte angeordneten Kamera;
- **Fig. 4**: ein Flussdiagramm zur Veranschaulichung des erfindungsgemäßen Verfahrens;
- **Fig. 5**: eine Darstellung zur Veranschaulichung der Übertragung von drei Signalen über zwei Leitungen (Kanäle);
- **Fig. 6**: eine Darstellung zur Veranschaulichung der Übertragung von drei Signalen über eine Leitung (Kanal).

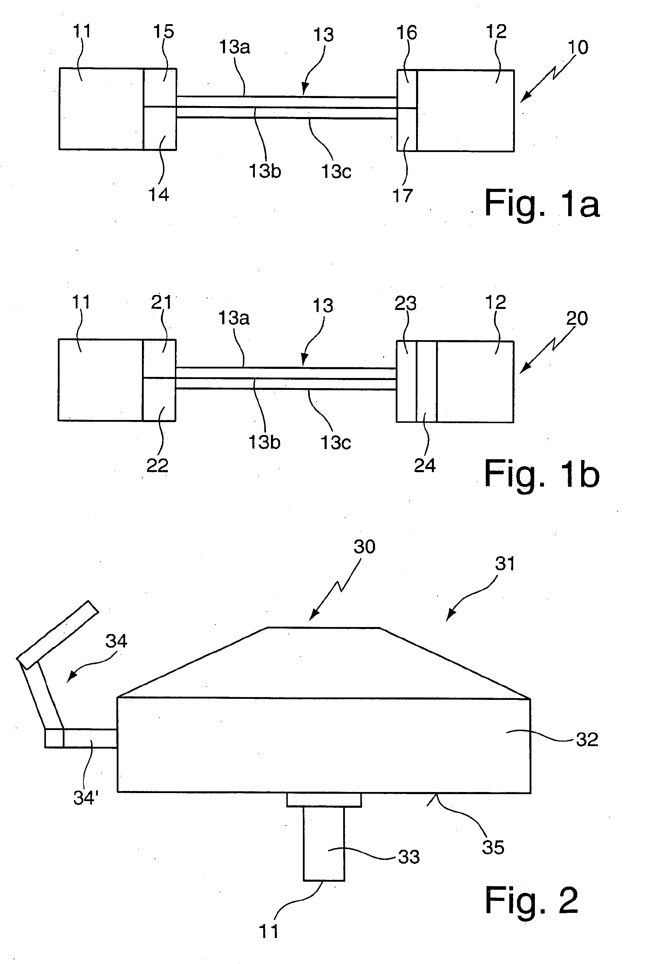

In der Fig. 1a ist ein OP-Leuchtensystem 10 gezeigt, welches eine im Bereich einer Opterationsleuchte angeordneten Kamera 11 und ein Kamerasteuergerät 12 umfasst, wobei zwischen der Kamera 11 und dem Kamerasteuergerät 12 ein Übertragungssystem 13 vorgesehen ist, welches weniger Übertragungskanäle 13a, 13b aufweist als das durch die Kamera gelieferte Videosignal Signalkomponenten hat. Die Übertragungskanäle 13a, 13b sind jeweils als verdrilltes Leitungspaar ausgebildet. Parallel zu den Übertragungskanälen laufen in der Zeichnung zu einer Linie zusammengefasst die Leitungen 13c für die Stromversorgung der Kamera 11 und der in der Fig. 1a nicht dargestellten Operationsleuchte, sowie ein Schutzleiter.

Um dennoch eine Übertragung des Videosignals über das Übertragungssystem 13 zu ermöglichen, ist kameraseitig eine Einrichtung 14 zur Komprimierung zumindest einer Signalkomponente vorgesehen. Weiterhin ist eine Zeitmultiplexeinrichtung 15 vorgesehen, durch die komprimierte Signalkomponenten zeitversetzt an das Übertragungssystem 13 übergeben werden können. Steuergeräteseitig ist daher eine Zeitdemultiplexeinrichtung 16 und eine Einrichtung zum Dekomprimieren 17 vorgesehen, um die ursprünglichen Signalkomponenten wiederherstellen zu können. Über das Übertragungssystem 13 kann auch eine Signalkomponente unkomprimiert übertragen werden. Bei der Übertragung muss darauf geachtet werden, dass steuergerätseitig die unkomprimierte Signalkomponente und die komprimierten Signalkomponenten einander zugeordnet werden können, so dass das ursprüngliche Videosignal rekonstruiert werden kann. Die Komponenten 12, 16, 17 können mit einer Anzeigeeinrichtung, beispielsweise einem Monitor, verbunden sein oder in einen solchen integriert sein.

Bei der Ausgestaltung des OP-Leuchtensystems 20 gemäß Fig. 1b ist im Unterschied zur Ausgestaltung der Fig. 1a kameraseitig ein A/D-Wandler 21 vorgesehen, der aus einem analogen Videosignal ein digitales Signal generieren kann. Mit der als MPEG-Komprimiereinrichtung ausgebildeten Einrichtung 22 zum Komprimieren kann das so generierte digitale Videosignal komprimiert werden und dann an das Übertragungssystem 13 übergeben werden. Steuergeräteseitig ist eine entsprechende MPEG-Dekomprimiereinrichtung 23 vorgesehen, um das ursprüngliche digitale Videosignal wiederherstellen zu können. Je nachdem, welche Signalform zur weiteren Verarbeitung benötigt wird, kann außerdem ein D/A-Wandler 24 vorgesehen sein, um wieder ein analoges Signal zu generieren, falls das digitale Signal nicht verarbeitet werden kann.

Aus der Seitenansicht einer Operationsleuchte 31 (Fig. 2) des OP-Leuchtensystems 30 ist deren prinzipieller an sich bekannter Aufbau ersichtlich. Die Operationsleuchte 31 umfasst einen Leuchtenkörper 32, welcher in seinem Innenraum in der Fig. 2 nicht sichtbare Leuchtmittel aufweist. Der Leuchtenkörper 32 ist über ein in der Fig. 2 nicht vollständig gezeigtes, einen als Schwenkarm ausgebildeten Tragarm umfassendes Tragsystem 34 schwenkbar an einer stationären Halterung an einer Decke oder einer Wand eines Gebäudes oder einer mobilen Einheit befestigt. Der Tragarm ist aus mehreren über Gelenke miteinander verbundenen Tragarmabschnitten 34' aufgebaut. Ein mit der Operationsleuchte 31 fest verbundener Tragarmabschnitt 34' des Tragsystems 34 ist in der Fig. 2 lediglich angedeutet. Daher lässt sich die Operationsleuchte 31 in X-, Y-, Z-Richtung dreidimensional bewegen und schwenken. Ein an dem Leuchtenkörper 32 angebrachter Handgriff 33 ermöglicht die Positionierung der Operationsleuchte 31 an beliebiger Stelle über einem Operationstisch.

In den Tragarm des Tragsystems 34 ist das Übertragungssystem 13 integriert. Die Tragarmabschnitte 34' sind frei durchdrehbar, d.h. es gibt keinen Anschlag, der einer Relativbewegung zweier benachbarter Tragarmabschnitte 34' im Wege steht. In der gelenkigen Verbindung weist das Übertragungssystem 13 daher Schleifringe auf.

An der Unterseite 35 tritt Licht aus, um die Operationsstelle auszuleuchten. In den Handgriff 33 ist eine Kamera 11 integriert, so dass diese immer auf den ausgeleuchteten Bereich ausgerichtet ist.

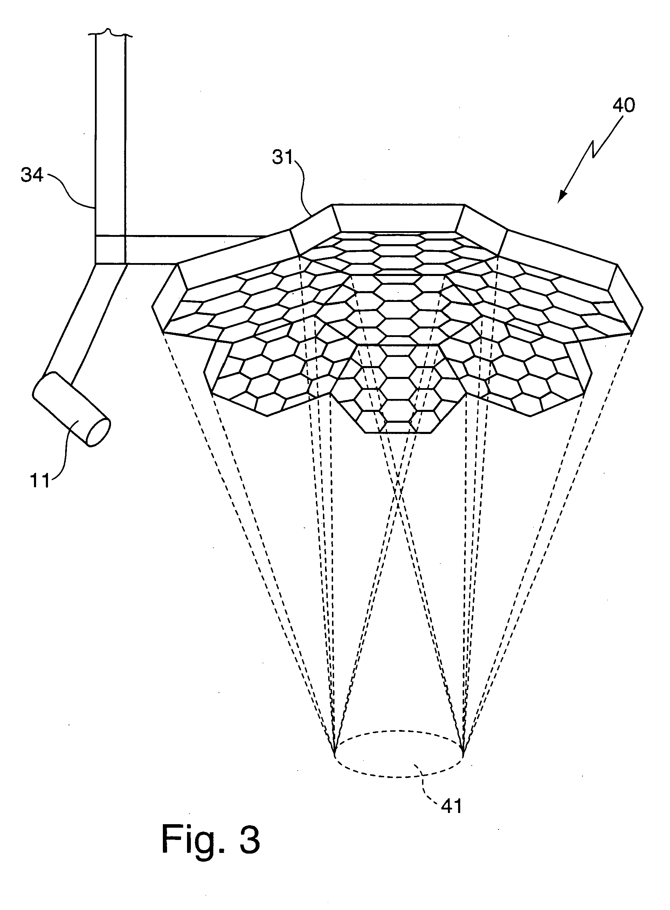

In der Fig. 3 ist ein OP-Leuchtensystem 40 gezeigt, das sich von demjenigen der Fig. 2 dadurch unterscheidet, dass die Kamera 11 an dem Tragsystem 34 neben der Operationsleuchte 31 angeordnet ist. Die Kamera 11 ist auf den durch die Operationsleuchte 31 ausgeleuchteten Bereich 41 ausgerichtet.

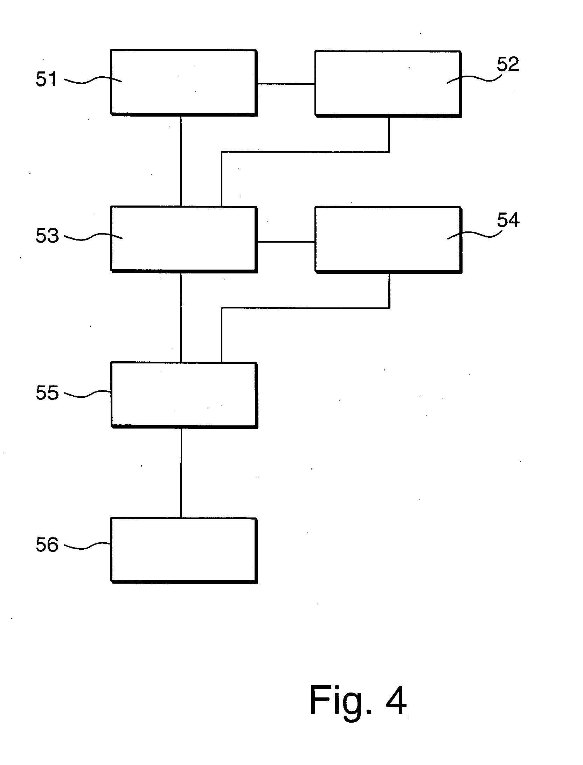

Anhand der Fig. 4 wird das erfindungsgemäße Verfahren erläutert. Im Verfahrensschritt 51 wird durch die Kamera 11 ein Videosignal, vorzugsweise ein analoges HDTV - Signal, bereitgestellt. Das analoge HDTV Signal wird entweder im Verfahrensschritt 52 in ein digitales Videosignal umgewandelt und dann einer zumindest teilweisen Komprimierung im Verfahrensschritt 53 unterzogen. Alternativ, wenn das analoge Videosignal bzw. Signalkomponenten des analogen Videosignals komprimiert werden sollen, können diese unmittelbar an den Verfahrensschritt 53 übergeben werden. Wenn ein oder mehrere analoge Signalkomponenten komprimiert werden, werden diese komprimierten Signalkomponenten im Verfahrensschritt 54 einem Zeitmultiplexverfahren unterworfen und dann an das Übertragungssystem 13 zur Übertragung gemäß Verfahrensschritt 55 übergeben. Handelt es sich um ein digitales komprimiertes Signal, so kann dieses unmittelbar an das Übertragungssystem zur Datenübertragung im Schritt 55 übergeben werden. Anschließend wird aus den komprimierten Signalen bzw. Signalkomponenten im Verfahrensschritt 56 das ursprüngliche Videosignal wiederhergestellt.

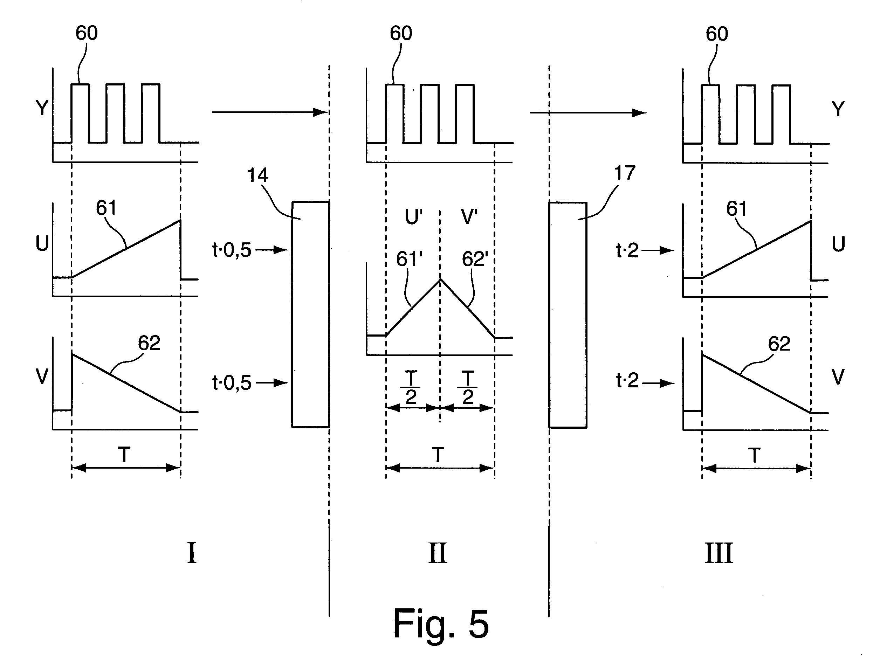

In der Figur 5 ist die Signalübertragung in drei Bereiche I, II, III unterteilt, wobei der Bereich I der Signalerzeugungsseite, beispielsweise der Kameraseite, entspricht, der Bereich II die Übertragungsstrecke mit im Ausführungsbeispiel zwei Kanälen darstellt, und der Bereich III der Signalempfangsseite, beispielsweise der Steuergeräteseite, entspricht. Das zu übertragende Signal umfasst die drei Signalkomponenten y, u, v, die während der Zeilendauer T beispielsweise die Signalformen 60 - 62 aufweisen. Die Signalkomponente y mit der Signalform 60 wird über einen ersten Kanal (Leitung) unverändert übertragen, so dass im Bereich III die Signalkomponente y mit der Signalform 60 vorhanden ist.

Die Signalkomponenten U, V werden der Einrichtung 14 zur Komprimierung zugeführt und die Zeit beispielsweise mit dem Faktor 0,5 multipliziert. Die komprimierte Signalkomponente U' erhält dann beispielsweise die Signalform 61' und die komprimierte Signalkomponente V' erhält beispielsweise die Signalform 62'. Die komprimierten Signalkomponenten U', V' werden jeweils während der halben Zeilendauer T/2 zeitversetzt zueinander (nacheinander) übertragen. Diese Übertragung erfolgt über denselben (zweiten) Kanal bzw. dieselbe Leitung. In der Dekomprimiereinrichtung 17 werden die Signalkomponenten U', V' wiederhergestellt, beispielsweise indem die Zeit der Signale 61', 62' mit dem Faktor 2 multipliziert wird, so dass die Signalkomponenten U, V mit ihrem ursprünglichen Signalverlauf 61,62 wiederhergestellt werden.

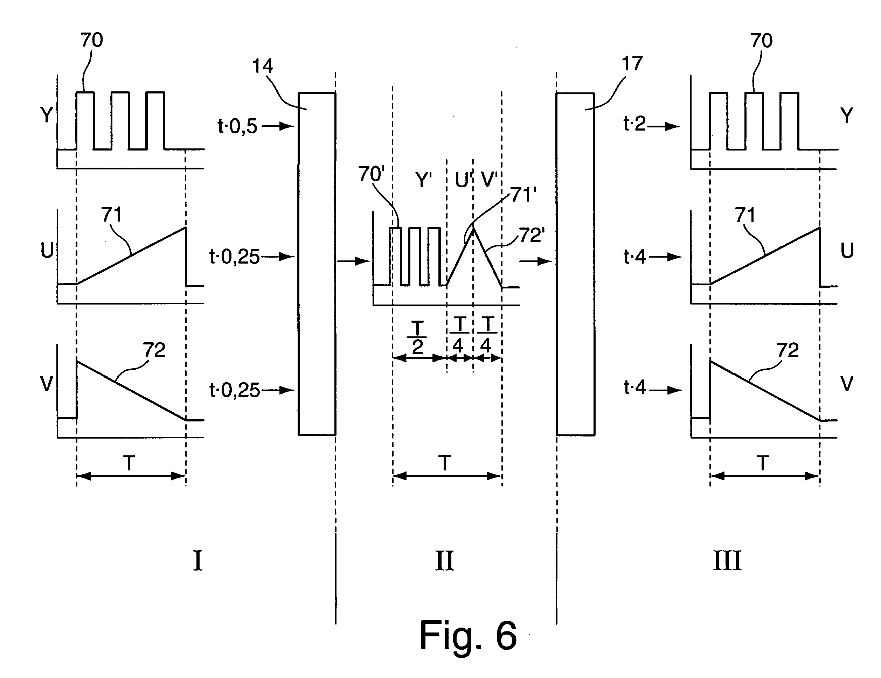

In der Figur 6 ist die Signalübertragung ebenfalls in drei Bereiche I, II, III unterteilt, wobei der Bereich I der Signalerzeugungsseite, beispielsweise der Kameraseite, entspricht, der Bereich II die Übertragungsstrecke mit im Ausführungsbeispiel einem Kanal darstellt, und der Bereich III der Signalempfangsseite, beispielsweise der Steuergeräteseite, entspricht. Das zu übertragende Signal umfasst die drei Signalkomponenten Y, U, V, die während der Zeilendauer T beispielsweise die Signalformen 70 - 72 aufweisen.

Die Signalkomponenten Y, U, V werden der Einrichtung 14 zur Komprimierung zugeführt, wobei beispielsweise die Zeit der Signalkomponente Y mit dem Faktor 0,5 und die Zeit der Signalkomponenten U, V mit dem Faktor 0,25 multipliziert werden. Die komprimierten Signalkomponenten Y', U', V' erhalten dann beispielsweise die Signalformen 70', 71' und 72'. Die komprimierten Signalkomponente Y' wird während der halben Zeilendauer T/2 und die komprimierten Signalkomponenten U', V' werden jeweils während einem Viertel der Zeilendauer T/4 zeitversetzt zueinander (nacheinander) übertragen. Die Übertragung erfolgt über denselben Kanal bzw. dieselbe Leitung. In der Dekomprimiereinrichtung 17 werden die Signalkomponenten Y, U, V wiederhergestellt, beispielsweise indem die Zeit der Signalform 70' mit dem Faktor 2 und die Zeit der Signalformen 71', 72' mit dem Faktor 4 multipliziert werden, so dass die Signalkomponenten Y, U, V mit ihrem ursprünglichen Signalverlauf 70, 71, 72 wiederhergestellt werden.

## Patentansprüche

1. Verfahren zur Signalübertragung eines Videosignals, insbesondere eines HDTV-Signals, bestehend aus mehreren Signalkomponenten (Y, U, V), über ein Übertragungssystem (13), wobei die Anzahl der Kanäle (13a, 13b) des Übertragungssystems (13) geringer ist als die Anzahl der Komponenten (Y, U, V) des Videosignals, mit den Verfahrensschritten:
a. Komprimieren zumindest einer Signalkomponente (Y, U, V);
b. Übertragen der zumindest einen komprimierten Signalkomponente (Y', U', V') über einen Kanal (13a, 13b) des Übertragungssystems (13);
c. Wiederherstellen des Videosignals.

2. Verfahren nach Anspruch 1, **dadurch gekennzeichnet, dass** zumindest zwei analoge Signalkomponenten (Y, U, V) komprimiert und über denselben Kanal (13a, 13b) übertragen werden.

3. Verfahren nach Anspruch 2, **dadurch gekennzeichnet, dass** die komprimierten Signalkomponenten (Y', U', V') zeit-gemultiplext übertragen werden.

4. Verfahren nach einem der Ansprüche 2 oder 3, **dadurch gekennzeichnet, dass** die komprimierten Signalkomponenten (Y', U', V') mit einer von der Anzahl der über denselben Kanal (13a, 13b) übertragenen Signalkomponenten (Y, U, V) abhängigen Geschwindigkeit an das Übertragungssystem (13) ausgegeben werden.

5. Verfahren nach einem der vorhergehenden Ansprüche, **dadurch gekennzeichnet, dass** eine Signalkomponente (Y, U, V) unkomprimiert über einen Kanal (13a, 13b) übertragen wird.

6. Verfahren nach einem der vorhergehenden Ansprüche, **dadurch gekennzeichnet, dass** aus zwei Signalkomponenten (Y, U, V) ein komprimiertes Videosignal generiert wird.

7. Verfahren nach Anspruch 1, **dadurch gekennzeichnet, dass** das Videosignal bzw. seine Signalkomponenten (Y, U, V) digitalisiert wird und ein digitales komprimiertes Signal erzeugt wird, das über einen Kanal übertragen wird.

8. Operations (OP)-Leuchtensystem (10, 20, 30, 40) mit zumindest einer Operationsleuchte (31) und einer an einem OP-Leuchten-Tragsystem (34) angeordneten Kamera (11), die ein Videosignal liefert, und einem mit der Kamera (11) über ein Übertragungssystem (13) verbundenen Kamerasteuergerät (12), wobei das Übertragungssystem (13) in dem Tragsystem (34) angeordnet ist, **dadurch gekennzeichnet, dass** an der Kamera (11) eine Einrichtung (14, 22) zur Komprimierung zumindest einer Signalkomponente des Videosignals vorgesehen ist.

9. OP-Leuchtensystem nach Anspruch 8, **dadurch gekennzeichnet, dass** das Tragsystem (34) zumindest zwei Tragarmabschnitte (34') aufweist, die durchdrehbar miteinander verbunden sind.

10. OP-Leuchtensystem nach einem der vorhergehenden Ansprüche 8 oder 9, **dadurch gekennzeichnet, dass** die Kamera (11) ein analoges HDTV-Signal, das drei Signalkomponenten (Y, U, V) aufweist, liefert.

11. OP-Leuchtensystem nach einem der vorhergehenden Ansprüche 8 bis 10, **dadurch gekennzeichnet, dass** kameraseitig eine Zeitmultiplexeinrichtung (15) vorgesehen ist.

12. OP-Leuchtensystem nach einem der vorhergehenden Ansprüche 8 bis 11, **dadurch gekennzeichnet, dass** steuergerätseitig eine Zeitdemultiplexeinrichtung (16) vorgesehen ist.

13. OP-Leuchtensystem nach einem der vorhergehenden Ansprüche 8 bis 12, **dadurch gekennzeichnet, dass** kameraseitig ein A/D-Wandler (21) und eine MPEG-Komprimiereinrichtung vorgesehen sind.
